Europäisches Patentamt

(19) European Patent Office

Office européen des brevets

(11) EP 0 787 462 A1

(12) EUROPEAN PATENT APPLICATION
published in accordance with Art. 158(3) EPC

(43) Date of publication:
06.08.1997 Bulletin 1997/32

(51) Int. Cl.$^6$: A61B 5/02

(86) International application number:
PCT/RU94/00016

(21) Application number: 94915716.8

(22) Date of filing: 02.02.1994

(87) International publication number:
WO 95/20907 (10.08.1995 Gazette 1995/34)

(84) Designated Contracting States:
AT BE CH DE DK ES FR GB GR IE IT LI LU MC NL PT SE

(71) Applicant:
TOVARISCHESTVO SOGRANICHENNOI OTVET STVENNOSTJU "RUSSKY TSENTR PERSPEKTI VNYKH TEKHNOLOGY" (RUSSINTSENTR)
Moscow, 127018 (RU)

(72) Inventors:
• RAGOZIN, Vyacheslav Nikolaevich
Moscow, 123424 (RU)
• DEGTYAREV, Vladimir Alexandrovich
Moscow, 123480 (RU)

(74) Representative: Ryan, Anne Mary
c/o Anne Ryan & Co.
60 Northumberland Road
Ballsbridge Dublin 4 (IE)

(54) OSCILLOMETRIC METHOD OF DETERMINING HAEMODYNAMIC INDICATORS IN THE ARTERIAL CIRCULATION OF A PATIENT AND MEASURING SYSTEM FOR APPLYING THE SAID METHOD

(57) The proposed oscillometric method of determining haemodynamic indicators in the arterial circulation of a patient involves recording pressure pulse waves (3) in a main vessel (1) which is subjected to linearly changing pressure (2) in a measurement pressure cuff (10). On the basis of this, a closed profile (ABCDLKFE) is formed during one measurement cycle, the profile consisting of the straight lines (A B), (B C), and (C D) in the diastolic part of the oscillometric curve (4) and of the lines (E F), (F K) and (K L) in the systolic part. The intersection points (B), (C), (F) and (K) indicate arterial pressure, while the amplitude values (Cc) and (Bb) of the pulse waves (3) in the profile define the geometrical parameters (dS) and (S) of the main artery (1) in question. The measurement system for applying thus method has a linear amplitude conversion characteristic and a rectilinear horizontal amplitude-frequency characteristic in a frequency range of between approximately 0 and 50 Hz across the entire signals conversion, amplification and recording channel.

FIG. 1

EP 0 787 462 A1

## Description

Field of the Invention

The present invention relates in general to medicine, more specifically to physiology and cardiology, and has particular reference to oscillometric methods for determining the hemodynamic parameters of the arterial portion of a patient's circulatory system and to measuring systems for carrying said methods into effect. The present invention can find application in clinical and experimental studies as a noninvasive method for diagnosis of the state of the circulatory system of a patient, e.g., for diagnosis and treatment of the hypotensive and hypertensive diseases, in circulatory insufficiency, and some other diseases of the cardiovascular system.

Background of the Invention

An ever-increasing incidence of cardiovascular diseases necessitates a rapid and reliable patients' examination for detecting and preventing even minute deviations from the norm of a healthy individual. With the aforesaid problem in view, an examination method is required, which is capable of yielding, for a short period of time, quite a number of parameters characteristic of the state of human circulatory system without causing any emotion stress therein that could affect the examination results. To this end, the method must be noninvasive and a negative effect exerted upon the patient be minimized.

The present-day state of the art knows quite a number of noninvasive methods for measuring the circulatory system paraneters. One of such methods developed under the agis of Pulse Metric, Inc. (i.e., 200 M DYNAPULSE TM instrument, its pamphlet being enclosed herein), consists in that an oscillometric curve of the arterial pulse is recorded by the measuring system, said curve resulting from the effect of a decreasing pressure in the cuff which builds up said pressure applied to the major arterial vessel under examination. The data thus obtained make it possible to measure the diastolic, systolic, and mean arterial pressure, as well as the pulse rate of the patient under examination.

The shape of an oscillometric curve recorded by said instrument demonstrates that the amplitude-frequency characteristics of the measuring system converts the volume pulse signals of the arterial pulse into signals close to the rate-of-change ones. As a result, formation of arterial pressure parameters is time-shifted and increased. But the key point consists in that the amplitude values of the pulse waves registered lose information about the volume being measured; therefore said amplitude values carrot be used for obtaining the data on, e.g., the cross-sectional area of the major arterial vessel under examination nor can one judge on the hydrodynamic characteristics of said vessel.

The oscillometric curve and the measurement results are displayed on the screen of a monitor, and the findings can be written in the memory of a personal computer or in the personal health diskette of the patient under examination. The measuring system, according to the method described above, comprises series interconnected a manually inflatable measuring cuff, a signal converter, amplifiers, and a logic circuit.

One more prior-art noninvasive method is known under the title of "Method and apparatus for determining blood pressure and cardiovascular condition" (cf. US Patent No.4,880,013). According to said method, the following three parameters are determined: the diastolic, the mean dynamic, and the systolic arterial pressure. When recording an oscillometric curve use is made of pressure reduction in the cuff, and the measuring system converts the volume signals of the pulse waves into signals close to the rate-of-change ones.

However, said method also fails to provide a reliable information about the arterial pressure parameters and on a change in the cross-sectional area of the large-caliber arterial vessel under examination.

The measuring system carrying said method into effect comprises a vessel-constricting pressure measuring cuff, a compression device, a converter of pneumatic signals into electric ones, amplification and logic electronic units series interconnected with one another.

Known in the present state of the art is also a tacho-oscillographic method for measuring arterial pressure as developed by N.N.Savitski (cf. his monography entitled "Biophysical fundamentals of circulation and clinical methods for studying hemodynamics", 1974, Leningrad, Meditsina PH, pp. 141 - 150 (in Russian). The method is also based on measurement of changes in the volume of the patient's limb which occur under the effect of a throbbing blood stream flowing along the major blood vessels. The method is capable of measuring the diastolic, mean dynamic, lateral systolic and end systolic arterial pressure in the major blood vessel of the limb to which a vessel-constricting cuff is applied. The arterial pressure parameters are determined while the pressure in the cuff is being raised, because it is believed that in this case linearity of pressure change can be attained more easily.

The measuring system for recording tacho-oscillograms comprises, according to said method, pneumatic units based on a vessel-constricting pressure measuring cuff, a receiver, and a converter of pneumatic signals into electric ones, as well as electronic amplification units and a recorder. An oscillo-metric curve is recorded while the pressure in the pressure measuring cuff is being raised, air being supplied to the cuff from the receiver.

The method discussed above, however, suffers from some methodical and instrumental errors.

The principal methodical (human) error of the tacho-oscillographic method resides in a subjective approach to decoding the data obtained in determining the signs of pressure. The amount of a total methodical error in absolute values cannot be evaluated, though practice demonstrates that the spread in the measured values of arterial pressure when decoding tacho-oscillograms ranges approximately from 10 to 15 mm Hg.

The basic instrumental error is determined by the measuring system and appears as a result of the linear conversion of the low-frequency portion (from 0 to 1.5 - 5 Hz) of a volume oscillometric signal sensed by the pressure measuring cuff from the major limb arterial vessel into rate-of-change signals. This results in a phase (time) shift of the arterial pressure paramaters during their formation on an oscillometric curve and hence in arterial pressure measurement errors.

Thus, none of the afore-discussed methods, when carried into effect with the aid of their own measuring systems, can represent real processes that occur in an arterial vessel under the effect of a variable pressure in the measuring cuff. In this case the signals being recorded carry more information about the rate of change of the volume of the major arterial vessel under examination. Thus, the error of determination of arterial pressure parameters is increased, the geometric parameters of the vessel involved and its hemodynamic characteristics cannot be calculated. Eventually, the amount of information obtained is decreased, whereby the diagnosis of the state of the circulatory system of the patient under examination as a whole becomes much less reliable.

Disclosure of the Invention

The present invention has for its principal object to provide such a noninvasive method for determining the hemodynamic parameters of the arterial portion of the circulatory system, of a patient and a measuring system for carrying said method into effect that makes it possible to obtain a wide range of highly reliable information characteristic of the state of the cardiovascular system of the patient under examination.

The foregoing object is accomplished due to the fact that in an oscillometric method for determining the hemodynamic parameters of the arterial portion of the circulatory system, consisting in that a vessel-constricting pressure measuring cuff is applied to the patient's major arterial vessel being examined, and an oscillometric curve of arterial pulse is registered in said cuff in the course of a pressure raise or drop therein, said oscillometric curve having the diastolic and systolic regions, as well as a graphic chart of a linear variation of the pressure applied to the cuff, according to the invention, said oscillometric curve of arterial pulse is recorded, with minimum amplitude and amplitude-frequency errors, within a frequency range of about 0 to 50 Hz, whereby said curve appears as a sequence of pulse pressure waves which represent a change in the volume of the major arterial vessel under examination with time under the action of a variable pressure in the vessel-constricting pressure measuring cuff, whereupon the extreme points are discriminated on the recorded oscillometric curve in the diastolic and systolic resions thereof, and straight lines are drawn through said extreme points until said lines intersect, thus producing a closed contour, after which the points of intersection of said straight lines are projected on the graphic chart representing a linear variation of the pressure applied, while ordinates of said points are in fact the arterial pressure characteristics.

This provides for measurement of real volumetric changes of the major arterial vessel being examined under the effect of a rising or falling pressure in the vessel-constricting pressure measuring cuff, the arterial pressure values and the geometrical dimensions of said vessel being determined or the basis of said volumetric changes of the vessel involved.

It is expedient to determine the cross-sectional areas of the major arterial vessel under examination in the systolic and diastolic phases against the amplitude values of said pulse waves of the oscillometric curve of the arterial pulse at the points of projection of the points of intersection of straight lines onto the graphic chart representing changes in the pressure applied.

It is reasonable that the linearly variable pressure be applied to the major arterial pressure under examination for about 40 - 60 seconds and that said pressure applied to the major arterial pressure under examination be increased or decreased in the same time lapse.

In the case of pressure raise the points of intersection of said straight lines may be projected consecutively onto the graphic chart representing a linear increase in the pressure applied so as to obtain at the points of their projections the respective absolute values of the diastolic arterial pressure, the mean dynamic arterial pressure, the lateral systolic arterial pressure, and the maximum systolic arterial pressure.

The value of the pulse arterial pressure may be determined as the difference between the values of the lateral arterial systolic pressure and and diastolic pressure, and the value of the hemodynamic stroke pressure be determined as the difference between the values of the lateral systolic arterial pressure and the maximum systolic arterial pressure.

It is practicable to measure the value of the pulse wave amplitude on the oscillometric curve of the arterial pulse at the point corresponding to said diastolic arterial pressure, said value being proportional to the increment of the cross-sectional area of the major arterial vessel under examination in the systolic phase.

It is possible to measure the value of the pulse wave amplitude on the oscillometric curve of the arterial pulse at the point corresponding to the mean dynamic arterial pressure, said value being proportional to the sum of the cross-sectional areas of the major arterial vessel under examination in the diastolic phase and its increment in the systolic phase, followed by subtracting the value of the thus-obtained increment of the cross-sectional area of said arterial vessel in the systolic phase from the thus-obtained total value of the cross-sectional areas of the major arterial vessel under examination, thus obtaining the cross-sectional area of said arterial vessel in the diastolic phase.

It is practicable to determine the deformation ratio of the major arterial vessel under examination as the ratio between the increment of the cross-sectional area of said arterial vessel in the systolic phase and said cross-sectional area of the same arterial vessel in the diastolic phase.

It is expedient that the bulk modulus of the major arterial vessel under examination be determined as the ratio between the product of the value of the pulse arterial pressure multiplied by the cross-sectional area of said arterial vessel in the diastolic phase, and the increment of the cross-sectional area of the same arterial vessel in the systolic phase, and that the velocity of the pulse wave propagation along the major arterial vessel under examination be determined as the ratio between the bulk modulus of said arterial vessel and the known blood density value raised to power 1/2.

It is possible to determine the blood flow velocity along the major arterial vessel under examination as the ratio between the value of the pulse arterial pressure and the value of the velocity of the pulse wave propagation along said arterial vessel multiplied by the known blood density value.

It is expedient that the estimated value of blood flow rate along the major arterial vessel under examination expressed in relative units, be determined as the product of a value proportional to the cross-sectional area of said arterial vessel in the diastolic phase, by the rate of blood flow along said arterial vessel, and that the blood minute volume expressed in relative units be determined as the product of the estimated rate of blood flow by 60 seconds.

The foregoing object is accomplished also due to the fact that a measuring system for determining the hemodynamic parameters of the arterial portion of the circulatory system comprises the following components interconnected in series to one another:

- a compression device aimed at effecting a linear pressure raise or fall in a vessel-constricting pressure measuring cuff, said device having a means for control of a calibrated value of said applied pressure;
- a converter of pneumatic pressure signals picked off said vessel-constricting pressure measuring cuff, into electric signals, said converter having its input connected to the output of the compression device and to the vessel-constricting pressure measuring cuff;
- a scaling amplifier having its input connected to the output of converter of pneumatic pressure signals and featuring a linear amplitude characteristic of conversion and a straight horizontal amplitude-frequency response within a frequency range of about 0 to 50 Hz, said amplifier being adapted for forming an oscillometric curve of the arterial pulse from signals picked off the vessel-constricting pressure measuring cuff;
- a recorder aimed at recording an oscillometric curve of the arterial pulse and having at least two channels one of which is connected to the output of a band-pass filter whose input, according to the invention, is connected to the output of the scaling amplifier of electric signals.

The measuring system comprises also an additional direct-current scaling amplifier of signals carrying the running values of the pressure effective in the vessel-constricting pressure measuring cuff, said amplifier having its input connected to the output of said converter of pneumatic signals and its output connected to the input of the second channel of the recorder.

Brief Description of the Drawings

In what follows the herein-proposed oscillometric method for determining the hemodynamic parameters of the arterial portion of the patient's circulatory system and measuring system carrying said method into effect are illustrated in some specific exemplary embodiments thereof to be read with reference to the accompanying drawings, wherein:

FIG.1 shows schematically a change in the cross-sectional area of the major arterial vessel under examination under the effect of a throbbing blood stream and of a linearly increasing pressure in the vessel-constricting pressure measuring cuff, as well as illustrates a volume oscillometric curve of arterial pulse obtained according to the present invention;

FIG.2 presents a real oscillometric curve of the arterial pulse of a particular patient N under examination, obtained according to the method proposed herein;

FIG.3 presents a real oscillometric curve of the arterial pulse of a particular patient K under examination, obtained according to the proposed method;

FIGS.4, 5, 6 present comparative data on the arterial pressure values obtained by the direct method, that is, by catheterization of the vessel being examined, and by the oscillometric method proposed herein;

FIG.7 illustrates a general block diagram of the measuring system, according to the invention; and

FIG.8 presents a real oscillometric curve and the running values of the pressure in the vessel-constricting pressure measuring cuff which are registered by the measuring system assembled according to the aforementioned block

4

diagram, according to the invention.

Best Method of Carrying Out the Invention

The herein-proposed oscillometric method for determining the principal parameters of the arterial portion of the patient's circulatory system is as follows.

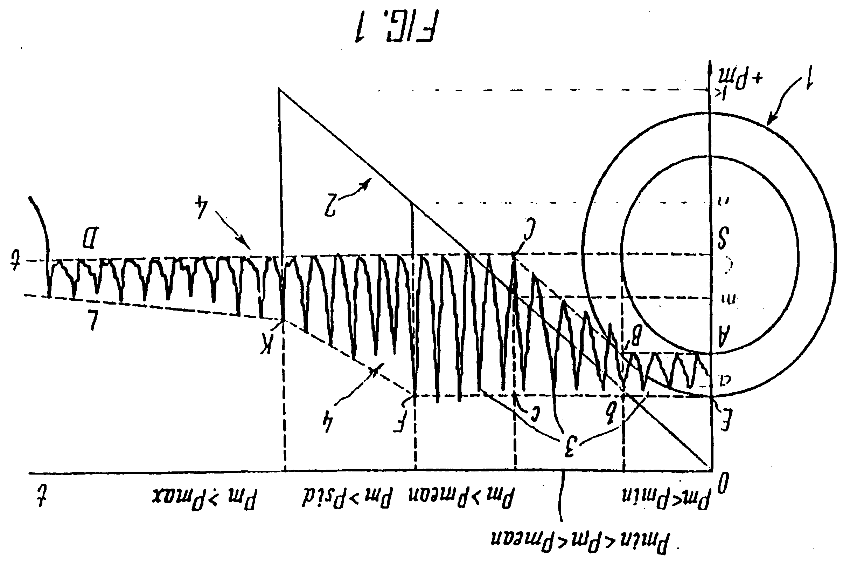

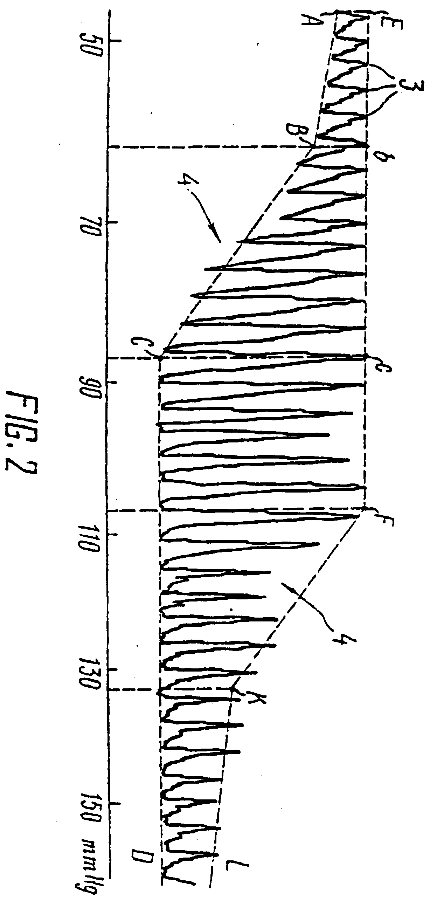

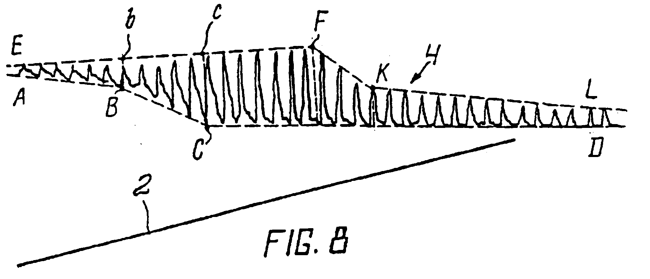

A pneumatic pressure cuff (omitted in FIG.1) is applied to a major arterial vessel 1 (FIG.1) under examination which runs along one of the limbs of the patient being examined, and a linearly variable pressure is built up in said cuff for about 40 - 60 seconds, said pressure varying approximately from 0 to 300 mm Hg (a straight line 2 in FIG.1). The signals of pulse waves 3 picked off the cuff are recorded, in the same period of time, with the aid of the measuring system, (FIG.7) featuring a linear conversion characteristic and a straight horizontal amplitude-frequency response in a frequency range of approximately from 0 to 50 Hz so as to obtain an oscillometric pulse 4 of the arterial pulse. The pulse waves 3 thus recorded represent a change in the volume of said arterial vessel 1 depending on the pulsating blood stream running therealong.

It is under the effect of a linearly rising pressure in the cuff 2 which is recorded in absolute values simultaneously with the signals of pressure of the pulse waves 3 that the oscillometric curve 4 of the arterial pulse is formed. Next the diastolic region indicated with the letters ABCD (FIG.1) and the systolic region indicated with the letters EFKL, after which extreme points are discriminated in said regions which belong to both of them, that is, the points ABCD in the diastolic region and the points EFKL in the systolic region. Next the straight lines AB, BC, and CD are drawn through said extreme points in the diastolic region and the straight lines EF, FK, and KL are drawn in the systolic region. The aforesaid straight lines are drawn until they intersect in said regions so as to obtain a virtually closed contour AEFKLDCBA bounded from left and right by the coordinate of the development time of the oscillometric curve 4. The points of intersection of the straight lines AB, BC, and DC, as well as those of the straight lines EF, FK, and KL correspond to the arterial pressure parameters being measured.

The principle of measurement of the arterial pressure parameters is based on comparison of the instantaneous values of the pressure in the vessel 1 under examination and of the increasing pressure (the straight line 2) in the vessel-constricting pressure measuring cuff, the absolute values of the latter pressure being recorded simultaneously on the oscillometric curve 4 of the arterial pulse. The parameters of of an arterial pressure that appear on the curve 4 in the course of one measuring cycle are projected onto the running values of the pressure effective in the cuff (that is, onto the straight line 2), and the values of said parameters are determined at the points a, m, n, and k of their projections.

The meaning proper of volume registration of the oscillometric curve 4 consists in the nature of signals converted by the pressure measuring cuff, because the pulse waves 3 are nothing but the values of the increment $dS$ of the cross-sectional area of the vessel 1 that appear in the systolic phase, multiplied by the length $L$ of the vessel constricted by the cuff, that is, the increment value of the volume of the major arterial vessel 1 under examination equals $dV = dS \cdot L$. The volume $V$ of said vessel 1 in the diastolic phase may be represented as the area $S$ of its cross section multiplied by the length $L$, that is, $V = S \cdot L$. FIG.1 shows schematically a cross-sectional view of the major arterial vessel 1.

Because the vessel length $L$ remains virtually invariable throughout the entire measuring cycle, the amplitude of the pulse waves 3 in the oscillometric curve 4 is directly proportional to the varying cross-sectional area $S$ of the vessel under examination.

FIG.1 illustrates schematically how the volume oscillometric curve 4 of the arterial pulse is formed, showing that the regular formation of the signs of arterial pressure in said curve is directly concerned with a change in the volume $V$ of the major vessel 1 under examination.

As the pressure $P_m$ in the cuff starts to be raised, the patient's limb portion in which the vessel to be examined is located, is compressed, with the result that the amplitude of the pulse waves 3 is somewhat increased. Inasmuch as the blood pressure in the arterioles and minor vessels during the diastolic phase is lower than in the major vessel 1 during the same phase, formation of the oscillometric curve 4 occurs under the effect of the pulsating pressure in said vessel.

The pressure $P_m$ in the vessel--constricting pressure measuring cuff rises gradually until it reaches the value of the diastolic pressure $P_{min}$ in the arterial vessel involved. Thenceforth the diastolic portion of the pulse waves 3 deflects downwards, obeying the law of rising pressure in the cuff, because the pressure $P_m$ in the cuff prevents the vessel 1 from getting completely patent in the diastolic phase. However, the next systolic discharge increases the lumen of the vessel 1 to its previous dimensions $S+ dS$, because the value of the lateral systolic arterial pressure $P_{sid}$ exceeds the pressure $P_m$ in the cuff, that is, $P_{sid} > P_m$. With the pressure $P_m$ in the cuff (the straight line 2) varying linearly, the ends of the diastolic portions of the pulse waves 3 "fall upon" the straight line (BC in FIG.1) which deflects downwards in compliance with the law of rising pressure in the cuff.

Once the pressure $P_m$ in the cuff has reached the value of the mean dynamic arterial pressure $P_{mean}$, i.e., $P_m > P_{mean}$ and starts exceeding it, the vessel 1 begins reducing its lumen at the end of the diastolic phase; hence no further decrease in the volume $V$ of the vessel 1 under the cuff occurs. The cross-sectional area $S$ of the vessel 1 under examination equals zero, which is indicated in the oscillometric curve 4 with a short-time flattening in the diastolic region ABCD which starts from the point C. Once a fresh blood portion has arrived at the beginning of a next systolic phase the lumen of the vessel 1 reaches its previous value at the pressure $P_{sid}$, because the pressure $P_m$ in the cuff is still lover than the lateral systolic pressure $P_{sid}$, i.e., $P_m < P_{sid}$. The first maximum pulse wave 3 in the oscillometric curve 4 of the arterial pulse corresponds to the instant mentioned above, said pulse wave being propertional to the sum of the cross-sectional areas S of the vessel 1 in the diastolic phase and the increment dS of the cross-sectional area S of said vessel 1 in the systolic phase, i.e., S + dS.

Further rise of the pressure $P_m$ in the cuff results in time extension of the aforesaid flattening in the diastolic region ABCD of the oscillometric curve 4, whereas the amplitudes of the pulse waves 3 retain their value until the pressure $P_m$ in the cuff reaches the value of the lateral systolic pressure $P_{sid}$ and are arranged on the straight line EF from the beginning of the measuring cycle. The ends of the diastolic sections of the pulse waves 3 in the diastolic region ABCD, srarting from the value of the mean dynamic arterial pressure $P_{mean}$ till the end of the measuring cycle, "fall upon" the straight line CD.

Once the pressure $P_m$ in the cuff has become equal to the lateral systolic pressure $P_{sid}$ in the vessel 1, that is, $P_m = P_{sid}$, and continues to rise, the amplitudes of the pulse waves 3 start decreasing which evidences of an incomplete opening of the major arterial vessel 1 under examination in the systolic phase, because the pressure $P_m$ in the cuff has already got as high as to prevent said process. With the pressure in the cuff varying linearly, the systolic portions of the pulse waves 3 are situated in the straight lire FK of the systolic region EFKL of the oscillometric curve 4.

As soon as the pressure $P_m$ in the cuff reaches the maximum value of the systolic pressure $P_{max}$, the arterial vessel 1 becomes nonpatent, blood stream along it ceases, tie pulse waves 3 caused by blood strokes against the proximal cuff edge are stabilized, their amplitude stops to be decreased rapidly, and the systolic portions thereof "fall upon" the straight line KL in the systolic region EFKL of the oscillometric curve 3.

The thus-obtained signs of arterial pressure indicated with the letters B, C, F, and K in the curve 3 are projected onto the chart 2 characteristic of a linear variation of the rising pressure in the cuff to obtain the arterial pressure values at the points a, m, n, and k of the projections of said signs as follows:

at the point a - the diastolic arterial pressure $P_m$;
at the point m - the mean dynamic arterial pressure $P_{mean}$;
at the point n - the lateral systolic arterial pressure $P_{sid}$;
at the point k - the maximum systolic pressure $P_{max}$.

Next the cross-sectional area S of the major arterial vessel 1 under examination in the systolic and diastolic phases is determined against the amplitude values of the pulse waves 3 in the oscillometric curve 4. To determine the increment dS of the cross-sectional area S of said vessel 1, the amplitude Bb of the pulse wave 3 is measured at the point B between the straight lines AB abd EF which bound the diastolic and systolic regions, respectively. Thereupon, to determine the total cross-sectional area dS + S of the major arterial vessel under examination, the amplitude Cc of the pulse wave 3 is measured at the point C between the straight lines CD and EF which bound the diastolic and systolic regions, respectively.

Further on, to find the cross-sectional area S of the majot arterial vessel 1 under examination in the diastolic phase, the value dS of increment of the cross-sectional area of the vessel 1 in the systolic phase is subtracted from the sum S + dS of the cross-sectional areas of said vessel.

Then the number of the pulse waves 3 are courted in the oscillometric curve 4 in the known period of time, thus determining the pulse rate $P_s$ of the patient under examination.

The remainder information is obtained is calculated using the known formulas of the circulation mechanics:

- the pulse arterial pressude dP is determined as the difference between the lateral systolic pressure $P_{sid}$ and the diastolic pressure $P_{min}$: $dP = P_{sid} - P_{min}$;
- the blood hemodynamic stroke pressure $P_{sp}$ is determined as the difference between the maximum systolic pressure $P_{max}$ and the lateral systolic pressure $P_{sid}$, i.e., $P_{sp} = P_{max} - P_{sid}$;
- the deformation ratio $\varepsilon$ is determined as the ratio between the increment dS of the cross-sectional area S of the major arterial vessel 1 under examination in the systolic phase and the cross-sectional area S of said vessel 1 in the diastolic phase, i.e., $\varepsilon = ds/S$;
- the bulk modulus K of the major arterial vessel 1 under examination is determined as the product of the pulse pressure dP by the cross-sectional area S of said vessel 1 in the diastolic phase divided by the increment dS of the cross-sectional area of said vessel 1 in the systolic phase:

$$K = \frac{dP \cdot S}{dS}$$

6

(The bulk modulus K of the major arterial vessel 1 under examination is in fact the reciprocal of the extensbility of said vessel, that is, $D = dS/S \cdot dP$ );

- the pulse wave velocity C is determined as the ratio between the bulk modulus K of the major arterial vessel 1 under examination and the known blood density value $\rho$ raised to power 1/2:

$$C = \sqrt{\frac{K}{\rho}} \; ;$$

- the blood velocity U along the major arterial vessel 1 under examination is determined as the ratio between the pulse pressure dP and the pulse wave propagation velocity C multiplied by the known blood density value $\rho$:

$$U = \frac{dP}{C \cdot \rho}$$

- the blood flow rate Q along the major arterial vessel 1 under examination is determined as the product of the blood velocity U by the cross-sectional area S of said vessel 1 in the diastolic phase: $Q = V \cdot S = dP \cdot S/Cp$ ;

Further calculations are carried out using the following formulas:

- the blood minute volume CO is determined as the rate Q of blood flow along the major arterial vessel 1 under examination per second multiplied by 60 seconds: $CO = Q \cdot 60$ ;
- the blood systolic discharge SV is determined as the value of the blood minute volume CO divided by the pulse rate $P_s$: $SV = CO/P_s$ .

The values of the blood flow rate Q, the blood minute volume CO, and the blood systolic discharge SV are calculated in terms of relative units which are used in monitoring the state of the examinee's cardiovascular system in the course of multiply repeated measurement of the hemodynamic parameters by the proposed oscillometric method. In such a case one must neither change the amplification factor of the measuring system nor remove the pressure measuring cuff. Time-dependent changes in the aforementioned parameters are determined in percent of the initial measurement.

For instance, examination of a patient aimed at selecting medicinal agents for treatment of hypertensive disease, may be carried out as follows:

- recording of an oscillometric curve of the arterial pulse;
- determining of hemodynamic parameters of the arterial system, that is, arterial pressure, bulk modulus K of the major arterial vessel 1 under examination, pulse wave propagation velocity C, and velocity of blood flow along said vessel 1 in absolute values, as well as the cross-sectional value S of said vessel 1 in the diastolic phase, blood flow rate $Q = S \cdot U$ , blood minute volume $CO = Q \cdot 60$ , and systolic blood discharge $SV = CO/P_s$ , wherein $P_s$ is the pulse rate, each of the thus-obtained relative values S, Q, CO, and SV are adopted to be 100%;
- a next recording and analysis of the oscillometric curve 4 following drug administration by the patient and commencement of its effect, without removing the pressure measuring cuff or changing the amplification factor of the recording equipment;
- determining of a shift of the hemodynamic parameters in absolute values and of the following parameters: cross-sectional area S of the major arterial vessel under examination, blood flow rate Q, blood minute volume CO, and systolic blood discharge SV expressed in percent of the previously obtained. It is by the time-dependent changes of the parameters thus measured that one can judge of the effect taken by the drug administered.

The aforedescribed law of development of the oscillometric curve 4 of the arterial pulse refers to an increase in the pressure $P_m$ in the cuff. When the pressure $P_m$ in the cuff decreases all the processes that proceed in the vessel 1 during formation of the oscillometric curve 4 run in a reverse sequence and hence the signs of the arterial pressure are formed in the reverse order, too. It is noteworthy that it is preferable to record the oscillometric curve 4 during an increase in the pressure $P_m$ in the cuff because even a high-rate increase of the pressure $P_m$ in the cuff followed by is slow decrease is in fact a load upon the vessel 1 under examination which modifies its tonus.

In what follows the method described above is illustrated by some specific examples of its realization.

Example 1

Male patient N., 45 was subjected to examination for the state of his cardiovascular system. The examination was conducted under outpatient clinical conditions with the patient in the recumbent position. The volume signals of the pulse waves of the oscillometric curve 4 and the signals of the increasing pressure $P_m$ in the vessel-constricting pressure measuring cuff were entered, through an analog-to-digital converter, into an IBM-compatible personal computer and were processed according to the method proposed herein.

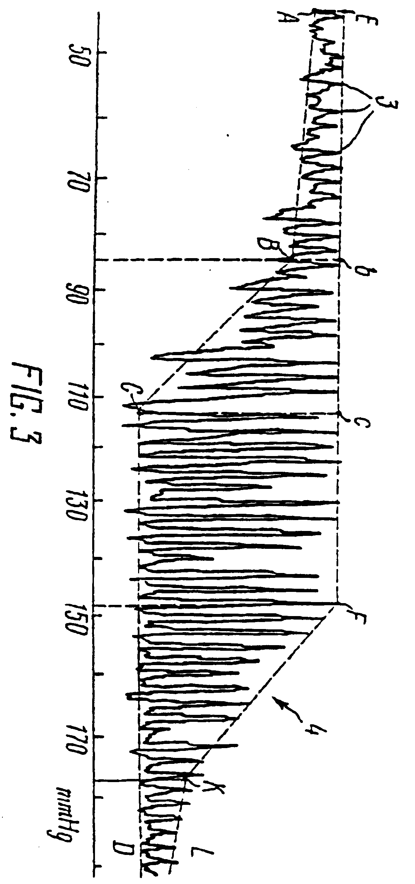

FIG.2 presents the oscillometric curve 4 of the arterial pressure taken from patient N. under examination during an increase in the cuff pressure. The running values of the cuff pressure $P_m$ are represented by the figures from 50 to 150 mm Hg situated under the oscillometric curve.

The thus-obtained oscillometric curve 4 is approximated by the straight lines AB, BC, and CD drawn through the points A, B, C, D in the diastolic region and by the straight lines EF, FK, KL drawn through the points E, F, K, L in the systolic region so as to obtain a closed contour ABCDLKFEA, where the points B, C, F, K of intersection of said straight lines are the signs of the arterial pressure. Then the aforesaid pointd are projected onto the running values of the pressure $P_m$ in the cuff to obtain the following pressure values:

- at the point B - the diastolic pressure $P_{min}$ equal to 61 mm Hg;
- at the point C - the mean dynamic arterial pressure $P_{mean}$ equal to 87 mm Hg;
- at the point P - the lateral systolic arterial pressure $P_{sid}$ equal to 106 mm Hg;
- at the point K - the maximum systolic arterial pressure $P_{max}$ equal to 133 mm Hg.

The pulse rate counted for a known period of time equals $P_s$ = 65 beat/min.

Then the pulse pressure ($dp = P_{sid} - P_{min}$) is calculated, which is equal in this particular case to 45 mm Hg, and the hemodynamic stroke pressure ($P_{sp} = P_{max} - P_{sid}$) equal to 27 mm Hg.

Thereupon the amplitude of the pulse wave 3 at the point B of intersection of the straight lines AB and BC is measured, which corresponds to the diastolic arterial pressure parameter. The value Bb of the amplitude of said wave situated between the point B and the point b that belongs to the straight line EF in the systolic region is proportional to the increment dS of the cross-sectional area S of the arterial vessel under examination in the systolic phase, and is equal to 13 mm in this particular case.

The value of the amplitude of the pulse wave 3 at the point C located between the diastolic and systolic regions between the point C and c is measured in a similar way. The value of said amplitude is proportional to the sum of the following areas: dS which is the increment of the cross-sectional area of the vessel under examination in the systolic phase, and S which is the cross-sectional area of said vessel in the diastolic phase. In this particular case this sum is equal to 48 mm.

The value proportional to the cross-sectional area S of the major arterial vessel 1 under examination is found as the difference between the wave amplitudes Cc and Bb measured at the points C and B, respectively. In the Example considered herein this value equals 48 - 13 = 35 mm.

Next the bulk modulus K of the major arterial vessel under examination is calculated. To convert the pressure units in mm Hg into the conventional system of units adopted in medicine for assessing the elastic properties of blood vessels use is made of a commonly known factor of 1333 for conversion of mm Hg into din/cm$^2$:

$$K = \frac{1333 \cdot dP \cdot S}{dS} = \frac{1333 \cdot 45 \cdot 35}{13} = 161,498 \text{ din/cm}^2.$$

The pulse wave propagation velocity C is equal to

$$\sqrt{K/\rho} = = 161,498/1.05 = 392 \text{ cm/s},$$

where $\rho$ is blood density normally equals approximately 1.05 kg/m$^3$.

The blood velocity U along the vessel under examination is:

$$U = \frac{1333 \cdot dP}{C} = \frac{13333 \cdot 45}{392 \cdot 1.05} = 145.7 \text{ cm/s}.$$

A conclusion can be drawn on the grounds of the examination performed that the principal parameters of the arterial portion of the patient's circulatory system are within the limits of norm for a healthy human being.

Example 2

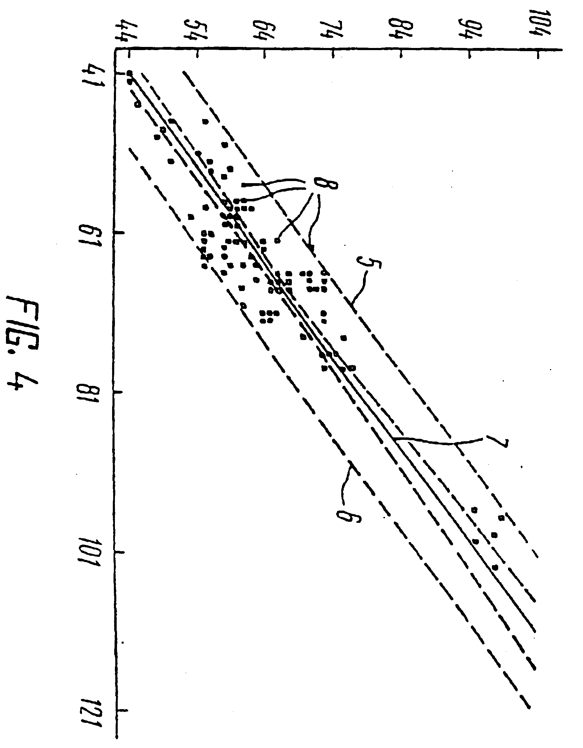

As a second example there may be adduced the data of a similar examination of male patient K., aged 62. FIG.3 shows the oscillometric curve 4 of the arterial pulse recorded in patient K. assuming the recumbent position, during an increase of the pressure $P_m$ in the cuff.

Like in the preceding Example, the oscillometric curve is approximated by the straight lines that form the closed contour ABCDLKFEA. The oscillometric curve thus recorded demonstrates that the patient suffers from cardiac arrhythmia. As a result, the pulse waves 3 have an unstable amplitude; therefore when determining the arterial pressure parameters by approximating the oscillometric curve 4 by the closed contour ABCDLKFEA one must adhere to the law of formation of an oscillometric curve by approximating the individual portions of said curve by straight lines.

Given below are the examination results:

Diastolic arterial pressure $P_{min}$= 85 mm Hg.
Mean dynamic arterial pressure $P_{mean}$= 112 mm Hg.
Lateral systolic arterial pressure $P_{sid}$ 148 mm Hg.
Maximum systolic arterial pressure $P_{max}$= 178 mm Hg.
Pulse arterial pressure dP = 63 mm Hg.
Hemodynamic stroke pressure $P_{sp}$= 30 mm Hg.
Deformation ratio of the major arterial vessel under examination dS/S = 0.32.
Bulk modulus K = 262,434 din/cm$^2$.
Velocity C of pulse cave propagation along the vessel under examination - 499.9 cm/s.
Blood velocity U along the vessel under examination - 160 cm/s.

An inference may be made on the grounds of both the examination results and the aspect of the oscillometric curve of the arterial pulse that the patient exhibits an abnormally high numerical value of the bulk modulus K and of the pulse wave propagation velocity C, said values pointing to a high rigidity of the major arterial vessel under examination. Too high arterial pressure values give evidence of the presence of the hypertensive disease in this patient.

The reliability of measurement of the arterial pressure parameters using the herein-proposed oscillometric method was determined by comparing, under clinical conditions, said method with the direct measurement method applied in contrast radiography examinations of the arterial portion of the cardiovascular system in humans exhibiting a considerable spread of the arterial pressure values. Use was made of the Cournand type catheter in such examinations, which was introduced into the Arteria brachialis as along the blood stream in said vessel. The position assumed by the catheter end at the proximal cuff edge was monitored radiologically. All the calculations concerning the results of comparison the data of indirect and direct measurements were carried out according to the relevant US standards (cf. American National Standard for Electronic or Automated Sphygmomanometers, Appendix B).

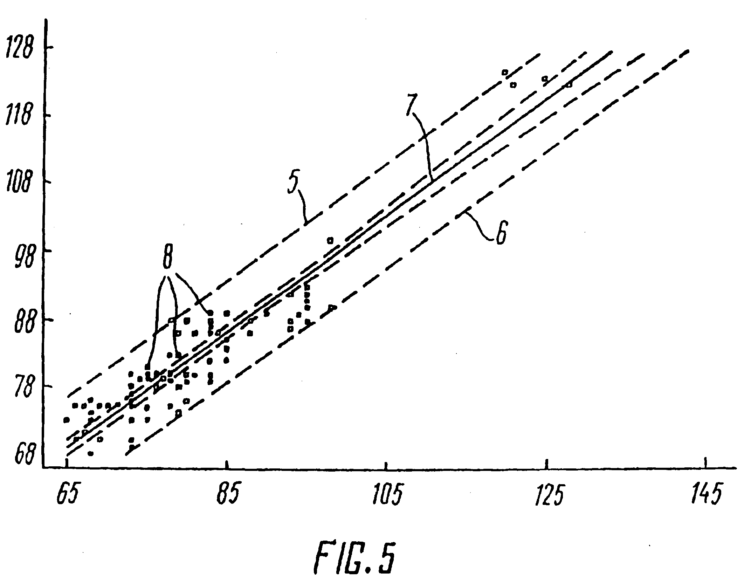

The graphic chart of FIG.4 represents the data on comparison of the values of the diastolic pressure $P_{min}$ obtained during simultaneous direct recording and by using the method proposed herein. The results of the direct measurement are plotted against the X-axis, the results of the oscillometric measurement being laid off on the Y-axis. Dotted lines 5 and 6 bound the zone of the confidence interval, a straight line 7 being the identification line. The results of each measurement are indicated with points 8 which are 102 in number in a given measuring cycle.

As can be seen from the graphic chart, the majority of the points 8 are concentrated at the identification line 7, the remainder points being within the limits of the confidence interval. The mean measurement error d = 0.535 mm Hg with the standard deviation sd = 4.336 mm Hg. The results of comparison demonstrate a high degree of reliability of the diastolic arterial pressure values $P_{min}$ measured by the non-invasive oscillometric method proposed herein.

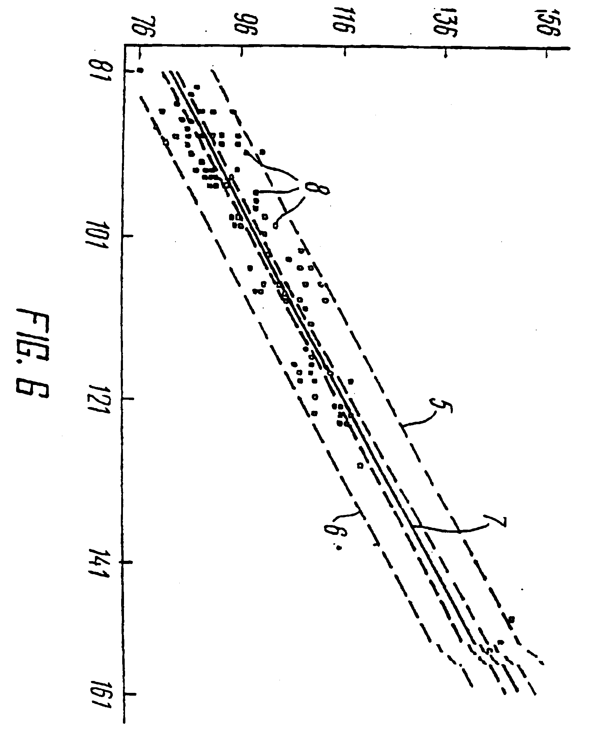

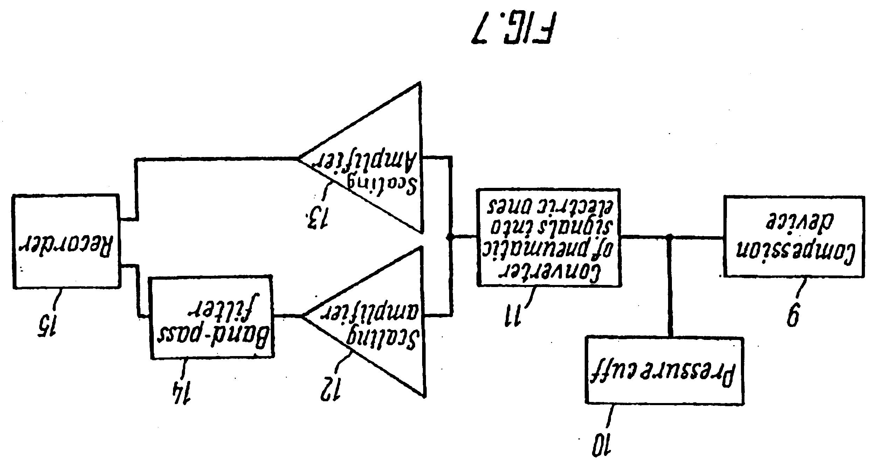

FIG.5 represents the graphic chart of the comparative data on the values of the mean dynamic arterial pressure $P_{mean}$, and FIG.6 is the graphic representation of a comparison of the systolic pressure $P_{sid}$ obtained by the direct measurement method, and that of the lateral systolic pressure obtained by the oscillometric method proposed herein. Use is made in these graphs of the same symbols as in the preceding ones.

The results of a comparison of the values given before which are presented in FIGS.5 and 6 also demonstrate a high degree of accuracy of the information about the values of the mean dynamic pressure $P_{mean}$ and of the lateral systolic arterial pressure obtained with the use of the oscillometric method proposed herein.

The method can find application in clinical practice in cardiologic departments for diagnosis, treatment, and prevention of cardiovascular diseases, as well as in resuscitation departments for obtaining information on the effect of the medicinal agents applied and on their administration doses. The method can successfully substitute the direct methods of arterial pressure measurement carried out currently and involving catheterization of the arterial portions of the circulatory system. The method also finds widespread application in preventive medical examinations and under domestic conditions, as well as in the practice of industrial medical establishments for diagnosis of the various forms of diseases and for their prevention which will substantially improve health of population. The method is also successfully applicable in aviation and space medicine for medical support of prolonged orbital flights, for preparation to such flights, and in the postflight period.

The principal requirement imposed on the measuring system for carrying into effect the oscillometric method for determining the hemodynemic parameters of the arterial portion of the circulatory system is the linearity of its amplitude--frequency response throughout the entire path of conversion, amplification, and recording of the pulse waves 3 of the arterial pulse during formation of the oscillometric curve 4 under the effect of the rising pressure 2 in, the vessel-constricting pressure measuring cuff. In addition, the amplitude-frequency response must be straight and horizontal in a frequency range of approximately from 0 to 50 Hz.

Another important requirement to the aforesaid measuring system is the linear characteristic of conversion of the arterial pulse signals along the entire path of conversion, amplification, and recording in the whole range of change of their amplitude.

One more requirement to the measuring system for carrying into effect the oscillometric method for determining the hemodynamic parameters of the arterial portion of the circulatory system is to provide a linear increase of the pressure in the vessel-constricting pressure measuring cuff approximately from 0 to 300 Hg for about 40 - 60 seconds.

The aforementioned requirements are but the principle ones that must be adhered to when developing and operating the measuring system for carrying the proposed oscillometric method into effect. It is only in such an instance that the oscillometric curve 4 of the arterial pulse carries the most reliable information about the real changes in the geometrical dimensions of the major arterial vessel 1 under examination, and formation of said oscillometric curve 4 obeys at all times the regularity described above.

The block-diagram of one of the embodiments of the measuring system for carrying said method into effect is illustrated in FIG.7

The system of recording the volume arterial oscillograms comprises series interconnected: a compression device 9 with a means for calibration and control over increasing and decreasing of the pressure in the vessel-constricting pressure measuring cuff 10; a converter 11 of pneumatic signals into electric ones; a first scaling amplifier 12 of oscillometric signals and a second scaling amplifier 13 of the signals of the running values of the pressure in the cuff 10; a band-pass filter 14 of the signals of the oscillometric curve 4 (FIG.1); and a recorder 15.

Used as the compression device 9 may be one of the widely known microcompressors which are made use of rather frequently in the systems of a similar purpose. Pressure can be raised in the cuff 10 either directly with the aid of such a microcompressor or after having preliminarily pumped up the receiver to a pressure two or three times the maximum patient's systolic arterial pressure. Operation of the microcompression device must not exert any influence upon conversion of pneumatic signals of the pulse waves 3 picked off the cuff 10.

Once a pressure has been applied to the measuring system, the pneumatic signals from the vessel-constricting pressure measuring cuff 10 are delivered to the pressure converter 11 to be converted there into electric signals corresponding to the arterial pulse, and into the signals representing the running value of the pressure in the cuff 10. Then said signals are amplified separately in two channels by the scaling amplifiers 12 and 13 of their own. The signals of the running value of the pressure in the vessel-constricting pressure measuring cuff 10 are applied at once to one of the channels of the recorder 15, and the signals representing the arterial pulse are first passed through the band-pass filter 14 and then applied to the other channel of the recorder 15. It is preferable that the signals be recorded in the orthogonal coordinates, e.g., on the mingograph available from the famous firm Siemens-Elema, or in a personal computer. Thus there are recorded in the course of one measuring cycle on the recorder 15 the oscillometric curve 4 of the arterial pulse and synchronously therewith the running values of the pressure 2 in the vessel-constricting pressure measuring cuff 10 which are subsequently analyzed.

Thus, the signals converted, amplified, and recorded with the use of the proposed measuring system differ virtually in nothing, as to their amplitude-frequency response, from real volume changes occurring in the major arterial vessel 1 under examination as a result of interaction of the throbbing blood stream in said vessel and an increasing pressure in the vessel-constricting pressure measuring cuff 10.

FIG.8 represents the oscillometric curve of the arterial pulse of the patient R., 60 recorded with the use of the aforedescribed measuring system.

As can be seen from the oscillometric curve 4 thus recorded, its formation in response to an increase of the pressure 2 in the vessel-constricting pressure measuring cuff 10 is accompanied by regular changes in the amplitude which represent a change in the volume V of the major arterial vessel 1 under examination. Next the oscillometric curve 4 is approximated by straight lines, whereupon said curve assumes the form of a contour, and the values of arterial pressure and the amplitudes of the pulse wave can be objectively measured at the points of inflection of said contour, said values being proportional to the cross-sectional area S of the major arterial vessel 1 under examination.

Industrial Applicability

The herein-proposed oscillometric method for determining the hemodynamic parameters of the arterial portion of patient's circulatory system and a measuring system for carrying said method into effect have found widespread application in the system of medical control of the crew members of the "Salyut" type manned space stations to provide extremely reliable measurement results.

Application of the proposed method for determining the hemodynamic parameters of the arterial portion of the circulatory system and of the measuring system for carrying said method into effect in public health services makes it possible to a great extent simplify and expedite population screening, to improve the quality of examination and the quantity of information thus obtained. The proposed method is used as the basis of a whole set of equipment aimed at noninvasive examination of not only arterial circulation in patients but also the circulatory system as a whole.

The noninvasive method for determining the hemodynamic parameters of the arterial portion of human circulatory system is physiologically substantiated and practically corroborated by a real recording of oscillometric curves and by comparison of the parameters thus obtained by the method proposed herein with those obtained by the direct methods of measurement.

The proposed method and the measuring system for carrying said method into effect have a number of great advantages over the methods in use nowadays.

First of all, a great amount of information taken off simply from a single cuff applied to the patient's limb major blood vessel under examination. In addition, nonskilled medical staff may be engaged in examination procedure, or even the patient being examined himself.

Used as the arterial pressure parameters are the regular processes proceeding in human organism and represented by the contour of the thus-formed oscillometric curve, the points of inflection of said contour resulting from from the effect of two oppositely applied pressures, that is, the intravascular pressure and the pressure exerted by the vessel-constricting pressure measuring cuff.

Determining of the hemodynamic parameters is based on the volumetric method for examining a major arterial vessel, which is carried into effect by the measuring system having the characteristics set forth herein.

The present method and the system for carrying it into effect enable one not only to determine the arterial pressure parameters but also to measure the dimensions of the vessel itself which are characteristic of the hemodynamics thereof.

By making use of the herein-proposed method one can obtain an information about more than 15 parameters of the arterial portion of the circulatory system of the patient under examination.

In addition, the oscillometric curve of virtually each of the patients being examined bears an individual nature and may therefore be utilized as the characteristic of the state of the patient's circulatory system.

Contour analysis of an oscllometric curve of the arterial pulse makes possible a complete automation of the processing of the results of measurements of the hemodynamic parameters of the arterial portion of the circulatory system in the patient under examination.

The measuring system for carrying the proposed method into effect is adequately simple and makes use of extensively known mechanical, pneumatic, and electronic units and components generally used for arterial pressure measurement in the modern equipment and apparatus.

## Claims

1. An oscillometric method for determining the hemodynamic parameters of the arterial portion of the circulatory system, consisting in that a vessel-constricting pressure measuring cuff is applied to the patient's major arterial vessel being examined, and an oscillometric curve of the arterial pulse is recorded in said cuff in the course of a pressure increase or decrease therein, said oscillometric curve having the diastolic and systolic regions, as well as a graphic chart of a linear variation of the pressure applied to the cuff, CHARACTERIZED in that an oscillometric curve (4) of the arterial pulse is recorded in a frequency range of about 0 to 50 Hz, whereby said curve appears as a sequence of pulse pressure waves (3) which represent a change in the volume of a major arterial vessel (1) under examination with time due to the effect of a variable pressure in a vessel-constricting pressure measuring cuff (10), whereupon extreme points (A, B, C, D and E, F, K, L) are discriminated on the recorded oscillometric curve (4) in the diastolic and systolic regions (ABCD, EFKL, respectively) thereof, and straight lines (AB, BC, CD and EF, FK, KL) are drawn through said extreme points until said lines intersect, thus obtaining a closed contour (AEFKLDCBA), after which the points (B, C, F, K) of intersection of said straight lines AB, BC, DC and EF, FK, KL are projected onto a graphic chart (2) representing linear changes of the pressure applied, whereas the ordinates (a, m, n, k) of said points are the arterial pressure characteristics.

2. An oscillometric method according to Claim 1 CHARACTERIZED in that the cross-sectional area (S) of the major arterial pressure (1) in the systolic and diastolic phases is determined against the amplitude values (Cc) and (Bb) of said pulse waves (3) of the oscillometric curve (4) of the arterial pulse at the points of projection of the points of intersection of the straight lines (AD, BC) and (BC, CD) onto the graphic chart (2) representing changes in the pressure applied.

3. An oscillometric method according to Claim 1, CHARACTERIZED in that the linearly variable pressure (2) is applied to the major arterial vessel (1) under examination for about 40 - 60 seconds.

4. An oscillometric method according to Claim 3, CHARACTERIZED in that the linearly variable pressure (2) applied to the major arterial vessel (1) under examination is increased or decreased in the period of time

5. An oscillometric method according to Claim 1, CHARACTERIZED in that with an increase in the pressure (2), the the points (B, C, F, K) of intersection of the straight lines (AB, BC), (BC, CD), and (EF, FK), (FK, KL) are consecutively projected onto the graphic chart (2) representing a linear increase in the pressure applied to obtain at the points (a, m, n, k) of their projections the respective absolute values of the diastolic arterial pressure ($P_{min}$), the mean dynamic arterial pressure ($P_{mean}$), the lateral systolic arterial pressure ($P_{sid}$), and the maximum systolic arterial pressure ($P_{max}$).

6. An oscillometric method according to Claim 5, CHARACTERIZED in that the value (dP) of the pulse arterial pressure is determined as the difference between the value ($P_{sid}$) of the lateral systolic arterial pressure and the value ($P_{min}$) of the diastolic arterial pressure.

7. An oscillometric method according to Claim 5, CHARACTERIZED in that the value ($P_{sp}$) of the blood hemodynamic stroke pressure is determined as the difference between the value ($P_{sid}$) of the lateral systolic arterial pressure and the value ($P_{max}$) of the maximum systolic arterial pressure.

8. An oscillometric method according to Claims 1 and 5, CHARACTERIZED in that the value of the pulse wave amplitude (Bb) on the oscillometric curve (4) of the arterial pulse at the point (B) corresponding to the diastolic arterial pressure ($P_{min}$) , said value being proportional to the increment (dS) of the cross-sectional area of the major arterial vessel (1) under examination in the systolic phase.

9. An oscillometric method according to Claims 1 and 5, CHARACTERIZED in that the value of pulse wave amplitude (Cc) on the oscillometric curve (4) of the arterial pulse is measured at the point (C) corresponding to the mean dynamic arterial pressure ($P_{mean}$), said value being proportional to the sum of the cross-sectional areas (dS + S) of the major arterial vessel (1) under examination in the diastolic phase (S) and the increment (dS) of said cross-sectional vessel area in the systolic phase, whereupon the value of the thus-obtained increment (dS) of the cross-sectional area of said major arterial vessel in the systolic phase is subtracted from the thus-obtained total value of the cross-sectional areas (dS + S) of the major arterial vessel (1) under examination, thus obtaining the cross-sectional area of said vessel (1) in the diastolic phase.

10. An oscillometric method according to Claim 9, CHARACTERIZED in that the deformation ratio $\varepsilon$ of the major arterial vessel (1) is determined as the ratio between the increment (dS) of the cross-sectional area of said major arterial vessel in the systolic phase and the cross-sectional area (S) of the same arterial vessel in the diastolic phase.

11. An oscillometric method according to Claim 9, CHARACTERIZED in that the bulk modulus (K) of the major arterial vessel (1) under examination is determined as the ratio between the product of the value (dP) of the pulse arterial pressure by the cross-sectional area (S) of said arterial pressure in the diastolic phase, and the increment (dS) of the cross-sectional area of the same arterial vessel in the systolic phase.

12. An oscillometric method according to Claim 11, CHARACTERIZED in that the velocity (C) of propagation of the pulse wave (3) along the major arterial pressure (1) under examination is determined as the ratio between the bulk modulus (K) of the same arterial vessel and the known blood density value ($\rho$) raised to power 1/2.

13. An oscillometric method according to Claim 12, CHARACTERIZED in that the velocity (U) of blood flow along the major arterial vessel (1) under examination is determined as the ration between the value of the pulse arterial pressure (dP) and the value of the velocity (C) of the pulse wave propagation along the same arterial vessel multiplied by the known blood density value ($\rho$).

14. An oscillometric method according to Claim 13, CHARACTERIZED in that the estimated value of the rate (Q) of blood flow along the major arterial vessel (1) under examination expressed in relative units, is determined as the product of the value proportional to the cross-sectional area (S) of the same arterial vessel in the diastolic phase by the rate (U) of blood flow along the same arterial vessel.

15. An oscillometric method accorfing to Clam 14, CHARACTERIZED in that the blood minute volume (CO) expressed in relative units is determined as the product of the estimated rate (Q) of blood flow by 60 seconds.

16. A measuring system for determining the hemodynamic parameters of the arterial portion of the circulatory system, comprising series-interconnected a compression device (9) aimed at effecting a linear increase or decrease of the pressure (2) in the vessel-constricting pressure measuring cuff (10), said device having a contrivance for control of a calibrated value of the pressure applied; a converter (11) of the pneumatic pressure signals picked off said vessel-constricting pressure measuring cuff (10) into electric signals, said converter having its input connected to the output of the compression device (9) and to the vessel-constricting pressure measuring cuff (10); a scaling amplifier (12) having its input connected to the output of said converter (11) of pneumatic signals; a recorder (15) aimed at recording the oscillometric curve (4) of the arterial pulse and having at least two channels one of which is connected to the output of a band-pass filter (14), CHARACTERIZED in that said band-pass filter (14) whose input is connected to the output of the scaling amplifier (12) of electric signals, features a linear amplitude characteristic of conversion and a straight horizontal amplitude-frequency response in a frequency range of about 0 to 50 Hz and is adapted for forming the oscillometric curve (4) of the arterial pulse from the signals picked off the vessel-constricting pressure measuring cuff (10), as well as in that said system comprises an additional scaling amplifier (13) of direct current, aimed at amplifying the running, values of the pressure in the vessel-constricting pressure measuring cuff (10), said amplifier having its input connected to the output of the converter (11) of pneumatic signals and its output connected to the input of the second channel of the recorder (15).

FIG. 1

FIG. 2

FIG. 3

FIG. 4

FIG. 5

EP 0 787 462 A1

FIG. 6

**FIG. 7**

Compression device — 9

Pressure cuff — 10

Converter of pneumatic signals into electric ones — 11

Scaling amplifier — 12

Scaling Amplifier — 13

Band-pass filter — 14

Recorder — 15

FIG. 8

## INTERNATIONAL SEARCH REPORT

| International application No. |
| --- |
| PCT/RU94/00016 |

**A. CLASSIFICATION OF SUBJECT MATTER**

Int. Cl.$^6$ : A61B 5/02

According to International Patent Classification (IPC) or to both national classification and IPC

**B. FIELDS SEARCHED**

Minimum documentation searched (classification system followed by classification symbols)

Int. Cl.$^6$ : A61B 5/00,5/02,6/06,6/021,6/022

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | SU, A, 1036318 (Bureau d'Etudes spécial de biocybernétique et cybernétique médicale), 23 August 1988 (23.08.88) | 1, 18 |
| A | SU, A1, 1308316 (Institut Radiotechnique de Taganrogsk) 7 May 1987 (07.05.87) | 1, 18 |
| A | SU, A1 1230588 (Bureau d'etudes spécial de biocy - bernétique et cybernétique médicale), 15 June 1988 (15.06.88) | 1 |
| A | US, A, 4484584 (Nippon Colin Co., Ltd), 27 November 1984 (27.11.84) | 1,16 |
| A | US, A, 4638810 (Criticon, Inc.), 27 January 1987 (27.01.87) | 1 |
| A | US, A, 4651747 (Baxter Travenol Laboratories), 24 March 1987 (24.03.87) | 1,16 |
| A | EP, A1, 0145208 (SEIKO INSTRUMENTS & ELECTRONICS LTD.) 19 June 1985 (19.06.85) | 1,16 |

[X] Further documents are listed in the continuation of Box C.    [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 9 September 1994 (09.09.94) | 27 October 1994 (27.10.94) |

| Name and mailing address of the ISA/ | Authorized officer |
| --- | --- |
| RU | |
| Facsimile No. | Telephone No. |

Form PCT/ISA/210 (second sheet) (July 1992)